# EUROPEAN PATENT SPECIFICATION

(11) **EP 1 908 418 B1**
(45) Date of publication and mention of the grant of the patent: **27.01.2010**
(21) Application number: 06021055.6
(22) Date of filing: 06.10.2006
(51) Int. Cl.: A61B 17/11, A61B 17/115, A61B 17/068

(54) **An anastomotic applier**
Anastomosenapplikator
Applicateur d'anastomose

(43) Date of publication of application: 09.04.2008
(73) Proprietor: ETHICON ENDO-SURGERY, INC., Cincinnati, OH 45242-2839 (US)
(72) Inventor: Thompson, Brian James, Cincinnati Ohio 45226 (US); Pastorelli, Alessandro, 00136 Roma (IT); Bilotti, Federico, 04011 Aprilia (Latina) (IT); Tacchino, Roberto, 00144 Roma (IT)
(74) Representative: Leihkauf, Steffen Falk

(56) References cited:
- EP-A1- 0 517 488
- WO-A-01/54594
- DE-B- 1 177 768
- US-A1- 2004 254 590

## Description

The present invention relates, in general, to devices for surgically modifying organs and vessels and more particularly to an applier for an anastomotic ring device for joining two organs or two parts of the same organ, such as the stomach and the small intestine and, within certain limits, blood vessels.

The percentage of the world population suffering from morbid obesity is steadily increasing. Severely obese persons are susceptible to increased risk of heart disease, stroke, diabetes, pulmonary disease, and accidents. Because of the effect of morbid obesity to the life of a patient, methods for treating morbid obesity are being researched.

Numerous non-operative therapies for morbid obesity have been tried with virtually no permanent success. Dietary counseling, behavior modification, wiring a patients jaws shut and pharmacologic methods have all been tried, and though temporarily effective, failed to correct the condition. Further, introducing an object in the stomach, such as an esophago-gastric balloon, to fill the stomach has also been used to treat the condition, however, such approaches tend to cause irritation to the stomach and are not effective long-term.

Surgical methods of treating morbid obesity have been increasingly used with greater success. These approaches may be generalized as those that reduce the effective size of the stomach, limiting the amount of food intake, and those that create malabsorption of the food that has been eaten. For instance, some patients benefit from adjustable gastric bands (AGB) that are advantageously laparoscopically placed about the stomach to form stoma of a desired size that allows food to fill an upper portion of the stomach, causing a feeling of satiety. To allow adjustment of the size of the stoma after implantation, a fluid conduit communicates between an inwardly presented fluid bladder of the AGB to a fluid injection port subcutaneously placed in front of the patients sternum. A syringe needle may then inject or withdraw fluid as desired to adjust the AGB.

Although an effective approach to obesity for some, other patients may find the lifestyle changes undesirable, necessitated by the restricted amount of food intake. In addition, the medical condition of the patient may suggest the need for a more permanent solution. To that end surgical approaches have been used to alter the portions of the stomach and/or small intestine available for digesting food. Current methods for performing an open or laparoscopic anastomosis for a gastric bypass include stapling, suturing and placing biofragmentable rings, each having significant challenges. For instance, suturing is time consuming, as well as being technique and dexterity dependent. Stapling requires placement of an anvil which is usually a large device difficult to introduce and remove after anastomosis.

Deployment of biofragmentable anastomotic rings requires sophisticated hand skill and is normally assisted by rigid applier devices which enable the surgeon to exert a compression force on the anastomotic ring device in order to connect the ring device with the tissue involved in the formation of the anastomosis. Such appliers are difficult to operate and the final result of the anastomosis is highly dependent on the reciprocal positioning between the ring device and the applier and between the single parts of the anastomotic ring device in case the ring device include different separate parts. Moreover, the deployment of an anastomotic ring device requires additional operational steps in order for instance to approximate the tissues involved in the anastomosis, create the anastomotic passage by pearcing, tissue widening or incision, as well as the withdrawal and transportation out of the body of the surgical instruments or parts of the anastomotic applier.

A prior art example of a surgical stapler is disclosed in WO 01/54594 A1 and an example of a known anastomic fastening ring is disclosed e.g. in EP 0 517 488 A1.

Document EP 0 517 488 A1 also discloses a surgical instrument for implanting this anastomotic fastening ring, which includes an anvil, an actuating member, a handle including an actuating mechanism and an elongate shaft connecting the handle with the actuating member for transmitting of tensile force for approximation of the ring parts.

Consequently, with reference to the formation of anastomoses in general and to surgical instruments for the placement of anastomotic ring devices in particular, there is a need to have at one's disposal an applier which overcomes the deficiencies of prior art instruments.

The aim of the present invention is therefore to provide a surgical instrument for implanting an anastomotic ring device in a laparoscopic or open surgery intervention having features which overcome the drawbacks cited with reference to the prior art.

Within the scope of the above aim, a particular aim of the present invention is to propose an anastomotic applier that incorporates a plurality of the above listed functions without increasing the complexity of the structure and handling of the applier itself.

A further aim of the present invention is to provide an anastomotic applier which enables the surgeon to create an anastomosis requiring less dexterity for the correct reciprocal positioning of the instrument parts and of the body tissue to be connected.

These aims are achieved by a surgical instrument for implanting an anastomotic ring device, comprising:
- a (preferably but not necessarily detachable) anvil including a first connecting part and a seat for receiving a distal compression ring of said anastomotic ring device and;
- an actuating member comprising:

- one or more seats for receiving a proximal compression ring with/and a retention element of said anastomotic ring device and;
- a second connecting part configured to engage the first connecting part for connecting the anvil with the actuating member, wherein the second connecting part is movable from a distal position in proximal direction in response to a tensile force, to approximate the anvil (and, hence, the distal compression ring) towards the actuating member (and, hence, the proximal compression ring) and;
- an actuating part configured to act on the retention element of the anastomotic ring device, the actuating part being movable from a proximal rest position in a distal direction, in response to a compressive force, for moving the retention element towards the anvil and in (preferably but not necessarily snap-) engagement with the distal compression ring to connect the compression rings;
- a handle including an actuating mechanism for producing said tensile approximation force and said compressive actuating force and;
- an elongate shaft connecting the handle with the actuating member, said shaft including means for transmitting said tensile approximation force to the second connecting part and said compressive actuating force to the actuating part.
   Thereby, the reciprocal positioning of the two compression rings, their reciprocal approximation which results in a controlled approximation of the corresponding tissues, and their ultimate connection through the retention element of the ring device are all incorporated in a single applier, permitting the relative operations to be performed in a well organized manner without negatively interfering one another.
   According to an aspect of the invention, the first connecting part of the detachable anvil and the second connecting part of the actuating member are suitable to snap-lock each other in a self aligning manner in order to simplify the connection and reciprocal positioning of the anvil and the actuating member resulting in a simplified self aligning reciprocal positioning of the distal compression ring and the proximal compression ring of the anastomotic ring device.
   According to a further aspect of the invention the anastomotic applier comprises also means for separating the distal compression ring from a central portion of the anvil having an outer diameter smaller than the inner diameter of the anastomotic compression rings, thereby enabling the remaining central portion of the anvil to be withdrawn in a proximal direction through the anastomotic orifice without being detached from the applier. These separating means can be embodied by a retractable holder configured to hold said distal compression ring and connect it to the anvil. The retractable holder may include a displaceable release portion, wherein a displacement of the release portion causes the retractable holder to retract inwardly in a radial direction and disconnect from the distal compression ring for enabling the anvil to be withdrawn through the center opening of the anastomotic ring device. In this context, the actuating member of the applier may include a releasing part configured to act on the release portion of the retractable holder, the releasing part being movable for displacing said release portion, in response to a release force preferably exerted by an actuating mechanism of the handle and transmitted through the elongate shaft. Alternatively, the anvil may include a holding part projecting radially outward from the anvil and configured to hold the distal compression ring for connecting it to the anvil, wherein a cutting edge of the actuating member is configured to cut away said projecting holding part for disconnecting the distal compression ring from the anvil and enabling the anvil to be withdrawn in through the center opening of the anastomotic ring device.
   According to a yet further aspect of the invention, the anvil comprises a tapered dilating portion having a diameter which increases in distal direction such that a distal and a proximal tissue disposed between the compression rings of the anastomotic ring device and preferably pierced through by the above mentioned first end second connecting portions can be dilated during approximation of the anvil towards the actuating member for creating the anastomotic lumen.
   Alternatively, such an anastomotic lumen can be created by a cutting edge of the actuating member movable in response to a cutting force for cutting the proximal and distal tissue compressed between the proximal and distal compression rings. The same cutting edge and the same incision operation may be advantageously used also to cut off the anvil from the projecting holding parts for separating the anvil from the distal compression ring.
   In accordance with a yet further aspect of the present invention, the actuating member of the anastomotic applier comprises a needle applier configured to hold a group of needles or staples for piercing and fixating the tissue held between the distal and proximal compression ring. The needle applier is movable for pushing the group of needles or staples into the anastomotic ring device, in response to a compressive needle force exerted by an actuating mechanism of the handle and transmitted by the elongate shaft.
   Advantageously, the elongate transmits the forces for the movement of the connecting parts, actuating part, release part, cutting edge by means of tensioning or compression members, which according to a preferred embodiment comprise tubular rods concentrically and slidingly arranged in one another, thereby realizing a very sturdy, reliable and compact anastomotic applier having a reduced size compared with the prior art instruments.
   These and other objects and advantages of the present invention shall be made apparent from the accompanying drawings and the description thereof, which illustrate embodiments of the invention and, together with the general description of the invention given above, and the detailed description of the embodiments given below, serve to explain the principles of the present invention.
- Figure 1 is a perspective view of an anastomotic applier equipped with an anastomotic ring device comprising a distal compression ring, a proximal compression ring and a retention element according to an embodiment of the invention.
- Figure 2 is an enlarged view of a detail of the anastomotic applier in figure 1.

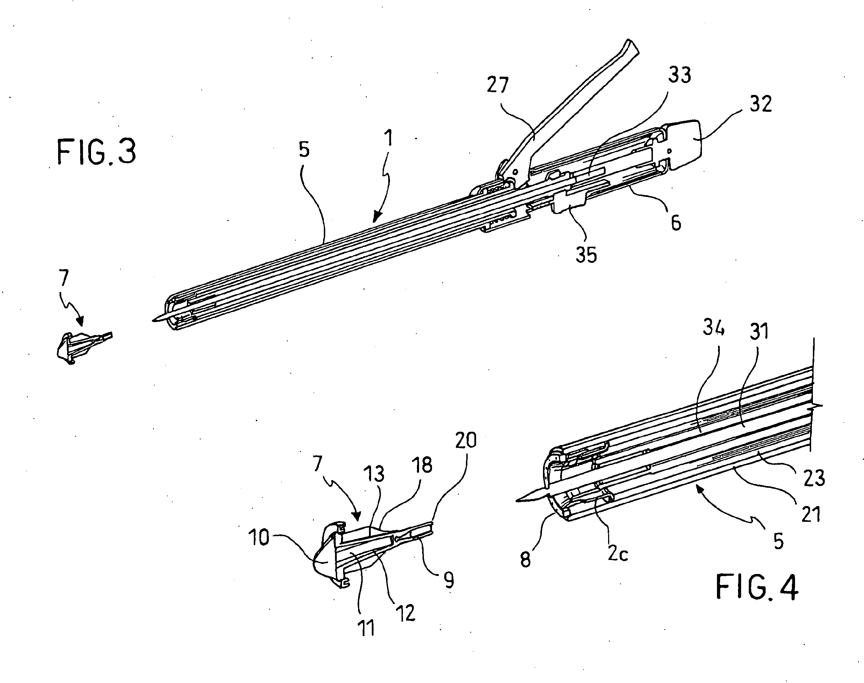

- Figure 3 is a longitudinal sectional view of the anastomotic applier in figure 1.
- Figure 4 is an enlarged view of a detail of the anastomotic applier in figure 3.

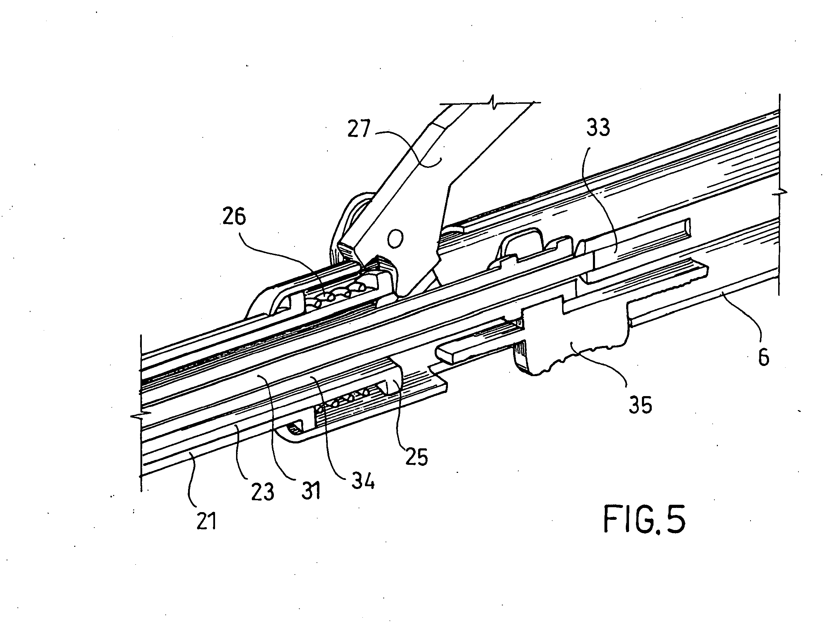

- Figure 5 is an enlarged view of a further detail of the anastomotic applier in figure 4.
- Figures 6 to 17 shows the anastomotic applier of figure 1 performing a sequence of operational steps during the implantation of the anastomotic ring device.
- Figures 18 and 19 are schematic perspective and partially sectional views of a detail of an anastomotic applier according to a further embodiment of the invention.

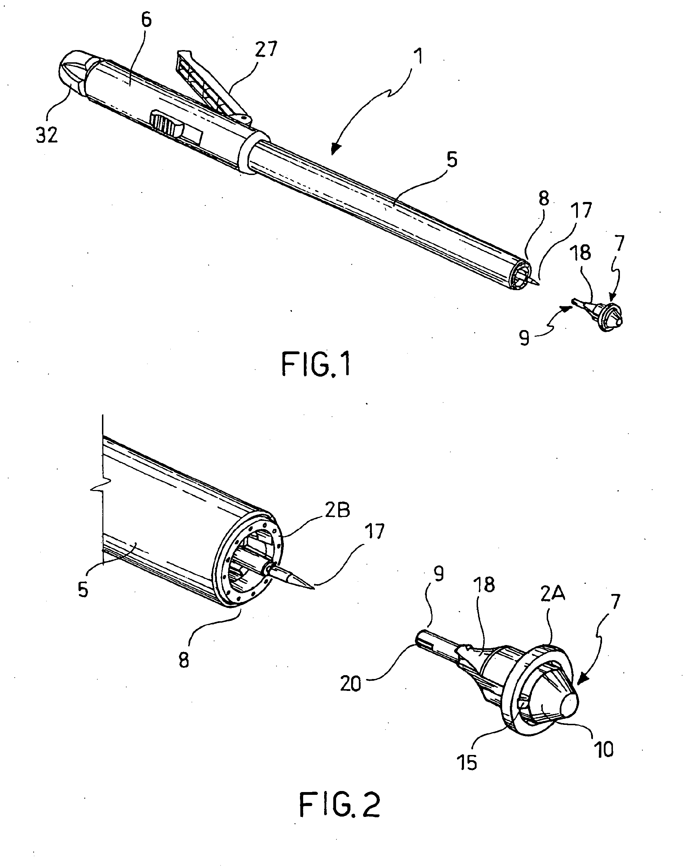

Referring to the drawings where like numerals denote like components throughout the several views, figure 1 depicts an applier 1 that advantageously laparoscopically, but also through a hand port or under open surgery conditions, or endoscopically if the dimension of the natural duct allows it, deploys and actuates an anastomotic ring device 2 having a distal compression ring 2A and a proximal compression ring 2B with a retention element or a separate retention element 2C suitable to snap connect the two compression rings 2A, 2B to another in order to form an anastomotic attachment between a distal tissue 3 (for instance intestinal wall tissue) and a proximal tissue 4 (for instance intestinal or gastric wall tissue) at an anastomosis target size, such as in a bariatric gastric bypass of a morbidly obese patient.

The applier 1 comprises an elongate insertion shaft 5, a handle 6 proximally connected with an elongate shaft 5 and a detachable anvil 7 connectable with an actuating member 8 of the applier arranged at the distal end of the elongate shaft 5.

The anvil 7 includes a first snap connecting part 9 for distally snap connect the anvil 7 in the anastomosis site with the actuating member 8 of the applier. The anvil 7 further defines a seat for receiving the distal compression ring 2A and holding it in position during the actuation of the anastomotic ring device 2. The seat may hold the distal compression ring 2A by press-fit, snap-fit, friction fit or shape fit.

According to one embodiment, the anvil 7 includes a distal, preferably conical, head 10 connected to a central rod 11 extending proximally from the head 10 and supporting a retractable holder 12. The anvil head 10 has an outer diameter less than the inner diameter of a central passage aperture of the anastomotic ring device formed by corresponding passage apertures of both compression rings 2A, 2B and the retention element 2C. The retractable holder 12 includes a radially deflectable or displaceable elongate release portion 13 with sloping release surfaces 14 and opposite holding teeth 15 extending radially outward from a distal end of the elongate release portion 13. The holding teeth 15 bear against a proximal bearing surface of the anvil head 10 and their ends protrude radially over the anvil head's outer periphery and are configured to engage corresponding holding surfaces, for instance one ore more circumferential grooves 16, of the distal compression ring 2A. A radial flexural displacement of the elongate release portion 13 causes the holding teeth 15 to retract radially inwardly and disconnect from the distal compression ring 2A for enabling the anvil head 10 together with the retracted holder 12 to be withdrawn in proximal direction through the center opening of the anastomotic ring device 2.

A proximal free end of the central rod 11 of the anvil forms a longitudinally slotted tubular seat with a substantially circumferential retaining edge forming the above mentioned first connecting part 9 suitable to snap receive a corresponding arrowhead shaped second connecting part 17 of the actuating member 8.

In accordance with an embodiment of the invention, the detachable anvil 7 comprises a tapered dilating portion 18 which extends proximally from the anvil head 10 and has a transverse dimension or diameter which increases in distal direction. The dilating portion 18 is preferably approximately cone-shaped and suitable to dilate the distal tissue 3 pierced by the anvil during approximation of the anvil towards the actuating member. In order not to interfere with the operation of the retractable holder 12, the truncated cone of the dilating portion 18 is arranged radially outside the elongate release portion 13 and separated from the latter by a circumferential gap which allows the access of a releasing part 19 of the actuating member to the release portion. Alternatively, the cone shaped dilating portion 18 is arranged around the elongate release portion 13 and comprises two opposite longitudinal grooves receiving the sloping release surfaces 14 of the retractable holder and exposing them to the action of the corresponding releasing part 19 of the actuating member.

Even though the anvil 7 could be inserted with its proximal first connecting part 9 through an aperture in the distal tissue 3 which has been previously pierced or incised by means of a trocar or scalpel separate from the anastomotic applier, according to a preferred embodiment of the invention, a trocar 20 is directly formed on or connected with the proximal end of the first connecting part 9 of the anvil and suitable to pierce through the distal tissue 3 in order to create an initial hole for the subsequent formation of the anastomotic orifice.

The elongate shaft 5 is preferably, but not necessarily rigid and substantially straight and includes an outer tube 21 having a distal end defining a seat 22 for receiving the proximal compression ring 2B and a proximal end connected with the handle 6. The seat 22 has a substantially circular shape with a size suitable to receive the proximal compression ring 2B with friction fit or snap fit in order to hold it in position during the insertion of the elongate shaft in the body of the patient and during the formation of the anastomosis. A substantially tubular actuating rod 23 is slidingly arranged inside the outer tube 21 and has a distal end defining a preferably annular pushing surface 24 which forms together with an internal cylindrical surface of the outer tube 21 a seat for slidingly receiving the retention element 2C which, in this embodiment, is separate from the proximal compression ring 2B. A proximal end of the actuating rod reaches inside the handle 6 and forms an annular flange 25. A compression spring element 26 is arranged between the flange 25 and a proximal end portion of the outer tube 21 or a spring seat especially formed in the handle, in order to elastically bias the tubular actuating rod 23 in proximal direction and maintain its proximal end in contact against a manual actuating trigger 27. The actuating trigger 27 is suitable for pushing the actuating rod 23 from a proximal rest position in distal direction for moving the retention element 2C towards and in snap engagement with the distal compression ring 2A to snap connect the compression rings 2A, 2B.

Preferably, the manual actuating trigger 27 includes a hinged trigger lever able to multiply the manually operated actuating force.

In accordance with an embodiment, the retention element 2C of the anastomotic ring device 2 comprises an annular proximal shoulder 28 suitable to engage a proximal surface of the proximal compression ring 2B and a longitudinal portion which distally protrudes from the proximal shoulder and forms elastically supported snapper teeth 29 extending radially outwardly from the longitudinal portion to enable snap engagement of the distal compression ring 2A. The longitudinal portion and the snapper teeth of the retention element 2C define slots or window openings 30 in alignment with the holding teeth 15 of the retractable holder 12 to enable the retention element 2C to be shifted in engagement with the distal compression ring 2A without interfering with the retractable holder 12.

The elongate insertion shaft 5 further houses a central approximating rod 31 which is slidingly received inside the outer tube 21 and has a distal end defining the above mentioned second connecting part 17. The second connecting part 17 is preferably arrow head shaped and configured to snap-engage the first connecting part 9 of the detachable anvil for connecting the anvil with the actuating member 8.

A proximal end of the central approximating rod 31 reaches inside the handle 6 where it is connected with a manual approximating knob 32 suitable for translating the approximating rod 31 from a distally advanced position in proximal direction to approximate the anvil carrying the distal compression ring towards the proximal compression ring. In accordance with a preferred embodiment, the approximating rod 31 is rotatably locked and the manual approximating knob 32 engages the its proximal end by means of a threaded connector 33 such that a rotation of the knob 32 causes the approximating rod 31 to translate along the threaded connector 33.

Even though the second connecting part 17 could be inserted through an aperture in the proximal tissue 3 which has been previously pierced or incised by means of a trocar or scalpel separate from the anastomotic applier, according to a preferred embodiment of the invention, a distal end of the second connecting part 17 has a trocar or a sharp tip formed or connected on it, suitable to pierce through the proximal tissue in order to create an initial hole for the subsequent formation of the anastomotic orifice.

The elongate shaft 5 further houses a preferably substantially tubular releasing rod 34 which is slidably arranged inside the outer tube 21 and has a distal end defining the above mentioned releasing part 19 (e.g. a tubular push portion or two oppositely arranged push pins positioned such as to be in alignment with the opposite longitudinal grooves of the dilating portion 18 of the anvil) and suitable to contact with pressure the sloping release surfaces 14 of the anvil's retractable holder 12 to retract the holding teeth 15 from the distal compression ring 2A.

A proximal end of the releasing rod 34 extends inside the handle 6 and is there connected with a manual releasing slider 35 suitable for pushing the releasing rod 34 from a proximal rest position in distal direction in order to retract the retractable holder. Advantageously, the manual release slider 35 includes a longitudinally adjustable sliding button directly connected with the proximal end of the release rod 34.

In accordance with a preferred embodiment, the central approximating rod 31 is slidingly arranged inside the tubular release rod 34 in order that the tensioned approximating rod guides the release rod 34 (which transmits a compression force) and increases its lateral stability.

In accordance with one embodiment of the invention, the distal and proximal tissue to be attached in anastomosis is held between the two compression rings 2A, 2B by the compressive force operated through their snap engagement. In this case the surfaces of the compression rings in contact with the tissue have preferably an enhanced roughness for increasing the friction between the anastomotic ring device and the tissue.

According to a further embodiment, the actuating member 8 of the anastomotic applier includes a needle applier configured to hold a group of needles or staples for piercing and fixating the tissue held between the distal and proximal compression ring. The needle applier is movable from a proximal rest position in distal direction, in response to a compressive needle force, for pushing the group of needles or staples into the anastomotic ring device. Such a needle force can be operated by a separate actuating mechanism and transmitted to the group of needles or staples by a separate pushing part or, which is preferable, the anastomotic applier 1 and the group of needles or staples are configured such that the needles can be applied by the same operation that causes the snap engagement of the two compression rings.

In a preferred embodiment, the retention element 2C of the anastomotic ring device itself defines a seat for receiving a group of needles 36 or staples such that, in response to a distal movement of the above mentioned actuating part, i.e. the pushing surface 24, the retention element 2C moves in snap engagement with the compression rings 2A, 2B and pushes contemporaneously the group of needles 36 or staples in the anastomotic ring device piercing the tissue 3, 4 held between the proximal and distal compression rings. In this case, the group of needles might advantageously comprise a self supporting needle ring which can be arranged on the annular shoulder 28 of the retention element 2C.

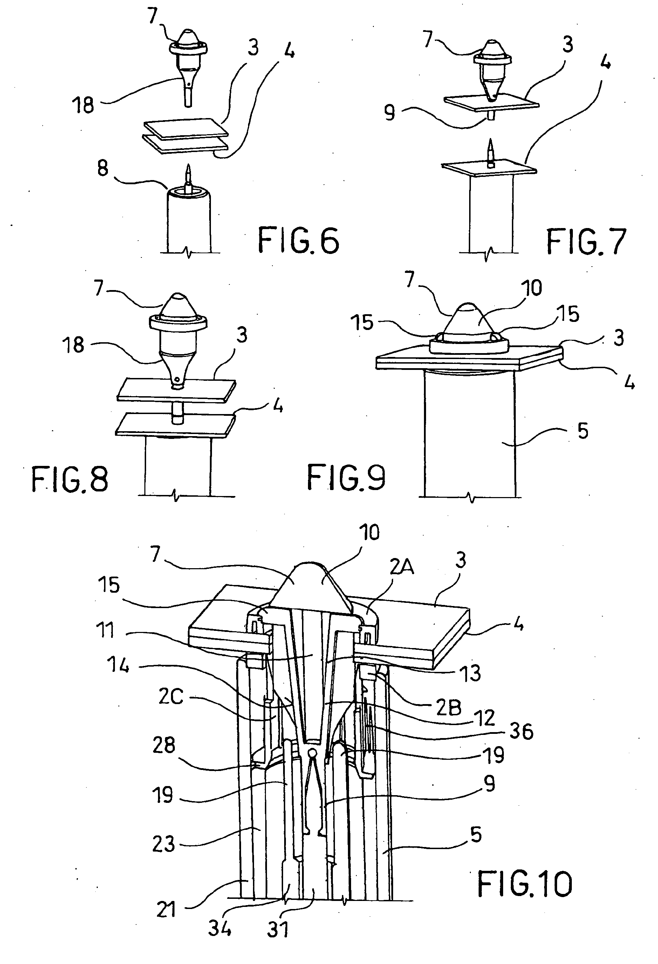

In use (figures 6 to 17), the detachable anvil is initially separate from the actuating member of the anastomotic applier 1 and can be positioned, for instance by means of surgical graspers or guide-devices (by laparoscopy, open surgery or through an endoscopic route) on the side of the tissue to be subject to anastomosis. Analogously, the actuating member 8 of the anastomotic applier is inserted in the body of the patient, e.g. through a trocar port leading to the proximal side of the proximal tissue to be subject to anastomosis. By pushing the anvil first connecting part against the distal tissue 3, the anvil trocar 20 will pierce the distal tissue defining an initial distal lumen for the anastomosis. Analogously, by pushing the sharp tip of the second connecting portion of the actuating member against the proximal tissue, a proximal initial lumen is created. The distal and proximal tissue can now be placed proximate to another and correctly aligned by moving the insertion shaft 5 towards the anvil 7 or vice versa and snap connect the two snap connecting parts 9, 17 to connect the anvil with the actuating member 8 of the applier (fig. 8). It is now possible to translate the anvil together with the distal compression ring proximally towards the first compression ring received on the distal tip of the actuating member. A controlled manual rotation of the approximating knob 32 translates the central approximating rod 31 proximally and applies a tensile force through the snap connecting parts 9, 17 to the anvil which approximates the two compression rings and clamps the distal and proximal tissue between them.

During the proximal translation of the anvil towards the actuating member, the increasing diameter of the cone shaped dilating portion 18 dilates the distal and proximal tissue to a diameter approximately equal to the inner diameter of the compression rings (fig. 10).

After the correct positioning of the distal and proximal compression rings and the widening of the anastomotic lumen, manual operation of the actuating trigger lever will produce a compression force which moves the actuating rod 23 and the push surface 24 distally to push the retention element 2C in (preferably but not necessarily snap-) engagement with the compression rings 2A, 2B connecting them.

Alternatively, the retention element might be provided with different coupling features, e.g. friction fit features, or screw on features.

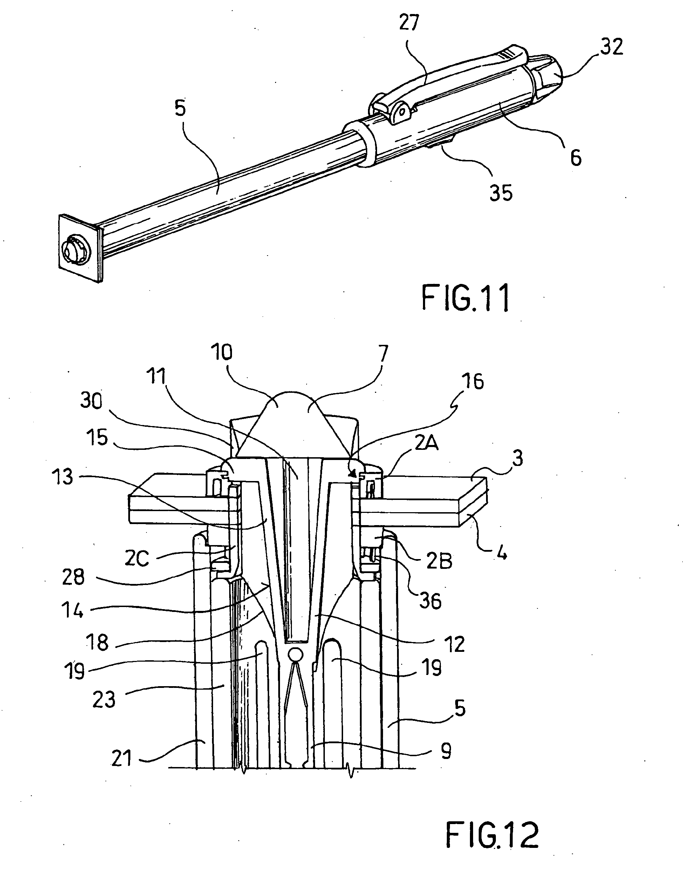

During the distal movement of the retention element, the needle ring 36 is pushed into both compression rings and pierces the rings of proximal and distal tissue clamped between them (fig. 12).

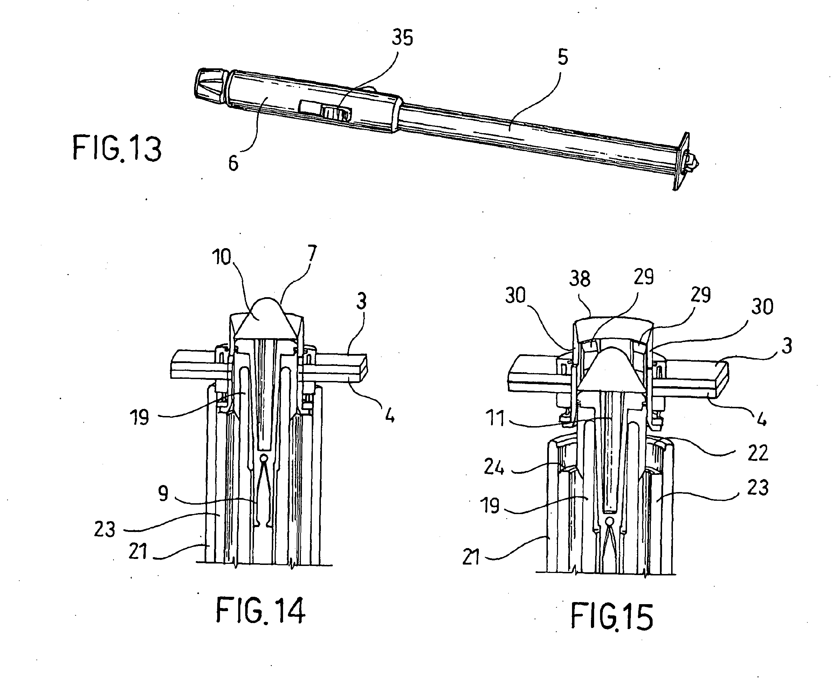

At this stage, the implantation of the anastomotic ring device is substantially completed. It is now necessary to remove the anastomotic applier from the body of the patient. To this end, manual shifting of the release slider 35 in distal direction will exert a compression force on the release rod and move it distally, causing the push pins 19 to contact with pressure the sloping release surfaces 14 of the retractable holder to radially displace the release portion 13 and, hence, disengage the holding teeth 15 from the distal compression ring 2A (fig. 14).

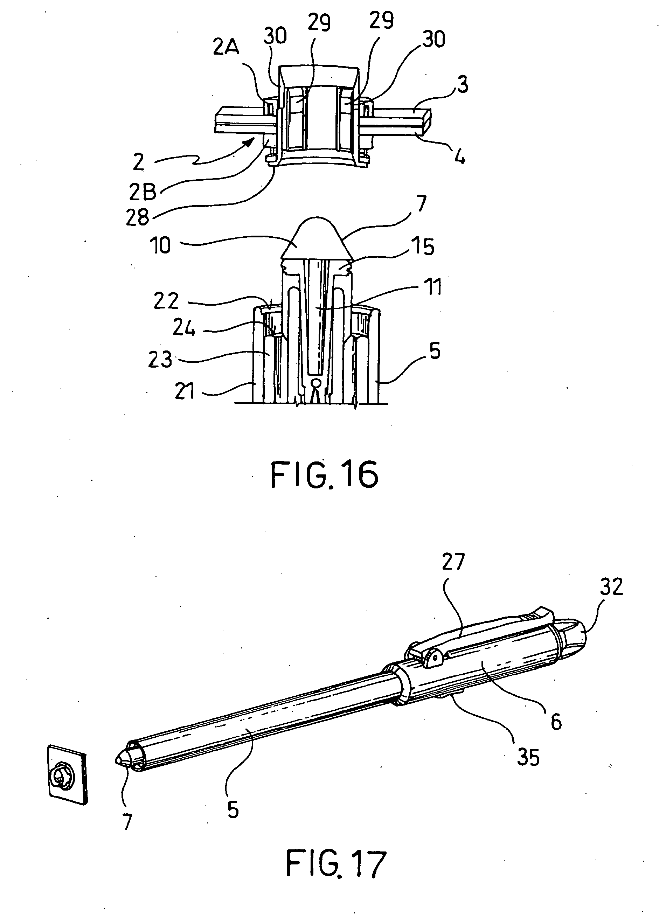

Once the holding teeth are retracted within a circumference smaller than the internal diameter of the anastomotic ring device, it is possible to withdraw the anvil, without detaching it from the applier, proximally through the anastomotic lumen. Before removing the applier from the anastomotic site, it might be necessary to disconnect the proximal compression ring 2B from the applier. This can be done, for instance by simply further distally advancing the push surface 24 with respect to the outer tube 21 in order to push the proximal compression ring out of the annular seat 22. The anastomotic applier can now be removed from the anastomosis (fig.16, 17) and taken out of the patient.

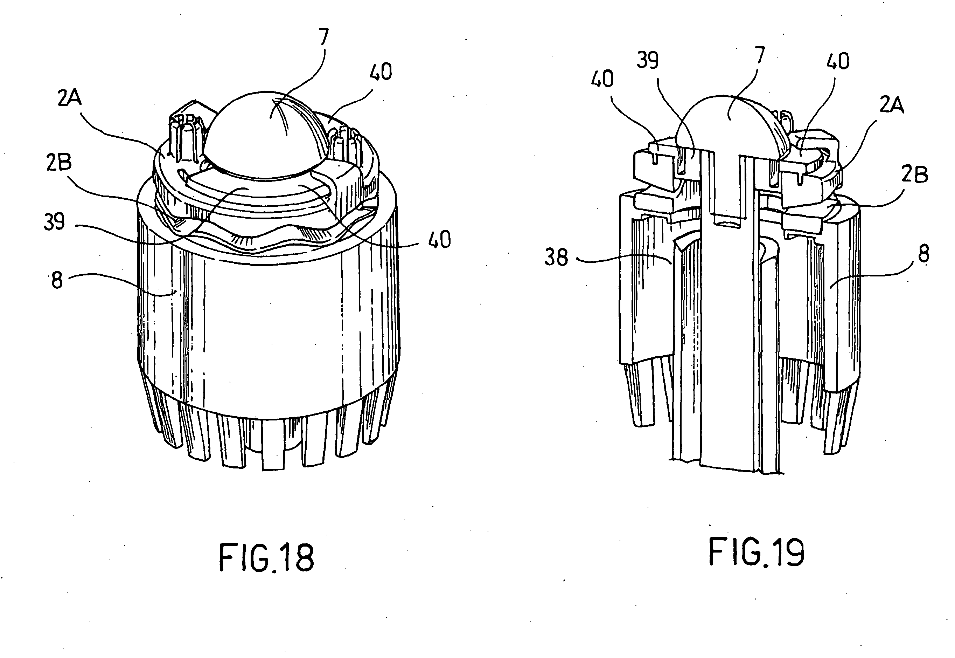

Figures 18 and 19 depict an alternative embodiment of the present invention, according to which the actuating member includes a cutting edge 38 movable from a proximal rest position in distal direction, in response to a compressive cutting force, for cutting the proximal and distal tissue clamped between the proximal and distal compression rings to create the anastomotic orifice. In this embodiment, the handle includes an actuating mechanism for producing said compressive cutting force and the elongate shaft includes means for transmitting said compressive cutting force to the cutting edge. Advantageously, the cutting edge might be operatively coupled with the actuating part 24 such that the cutting of the anastomotic lumen is performed contemporaneously with the snap engagement of the ring device or at least due to the same actuating force, obviating a separate cutting mechanism.

As can be seen from figures 18 and 19, the detachable anvil may comprise a cut washer 39 providing a cutting surface for the cutting edge 38 to press against in order to complete the cut. According to a further development of this embodiment, the cut washer 39 includes a holding part 40 projecting radially outward from the anvil and being configured to hold the distal compression ring 2A for connecting it to the anvil 7. The cutting edge 38 is configured to cut away said projecting holding part 40 for disconnecting the distal compression ring from the anvil and enabling the anvil to be withdrawn in proximal direction through the center opening of the anastomotic ring device.

Figures 18 and 19 show also an anastomotic ring device which differs slightly from that shown in the previous figures. In the present case, the retention element is integral with the proximal compression ring and the rings have a circumferentially wavy shape which consents the formation of anastomotic lumen having a larger diameter than the ring devices itself as the wavy shape of the tissues forming the anastomosis will flatten and circumferentially lengthen after removing of the bio-absorbable or bio-fragmentable compression rings. These features of the ring device itself can be readily applied also to the previously described embodiments. The respective seats for receiving the wavy compression rings are advantageously, but not necessarily, wavy, too.

Turning again to the cutting edge suitable to cut through the distal and proximal tissue walls forming the anastomosis, such a cutting edge 38 might be also formed on the distal end of the snapper portion 2C for cutting through residual tissue projecting in the anastomotic lumen after widening, which would otherwise hinder the insertion of the snapper portion.

While the present invention has been illustrated by description of several embodiments and while the illustrative embodiments have been described in considerable detail, it is not the intention to restrict or in any way limit the scope of the appended claims to such detail. Additional advantages and modifications may readily appear to those skilled in the art.

For instance, the elongate insertion shaft might be curved or flexible in order to allow access through a long lumen, such as a section of the small intestine. Accordingly, also the rods housed inside the elongate shaft for transmitting the approximation, actuation, release and cutting movements might be flexible in order to allow the flexion of the whole insertion shaft.

Preferred materials for the fabrication of the applier and its single components are biocompatible polymers and/or metals. Preferred materials for the anastomotic ring device are biocompatible and advantageously bio-fragmentable or bio-absorbable materials.

In order to better visualize the anastomotic site during deployment, the applier 1 might advantageously include illuminating means, comprising an illuminating power source and an electroluminescence device known in the art and therefore not shown in the figures.

## Claims

1. A surgical instrument (1) for implanting an anastomotic ring device (2), comprising:
- an anvil (7) including a first connecting part (9) and a seat (15) for receiving a distal compression ring (2A) of said anastomotic ring device (2) and;
- an actuating member (8) comprising:
- one or more seats (22) for receiving a proximal compression ring (2B) and a retention element (2C) of said anastomotic ring device (2) and;
- a second connecting part (17) configured to engage the first connecting part (9) for connecting the anvil (7) with the actuating member (8), wherein the second connecting part (17) is movable from a distal position in proximal direction in response to a tensile force, to approximate the anvil (7) towards the actuating member (8) and;
- an actuating part (24) configured to act on the retention element (2C) of the anastomotic ring device (2), the actuating part (24) being movable from a proximal rest position in a distal direction, in response to a compressive force, for moving the retention element (2C) towards and in engagement with the distal compression ring (2A) to connect the compression rings;
- a handle (6) including an actuating mechanism (32, 33, 25, 26, 27) for producing said tensile approximation force and said compressive actuating force and;
- an elongate shaft (5) connecting the handle (6) with the actuating member (8), said shaft including means for transmitting said tensile approximation force to the second connecting part (17) and said compressive actuating force to the actuating part (24).

2. A surgical instrument (1) according to claim 1, wherein said first connecting part (9) and said second connecting part (17) are suitable to snap-connect each other.

3. A surgical instrument (1) according to claim 1 or 2, wherein the anvil (7) comprises a retractable holder (12) configured to hold said distal compression ring (2A) and including a release portion (13), wherein a replacement of the release portion (13) causes the retractable holder (12) to retract radially inwardly and disconnect from the distal compression ring (2A) for enabling the anvil (7) to be withdrawn in proximal direction through the center openings of the anastomotic ring device (2),
wherein said actuating member (8) comprises a releasing part (19) configured to act on the release portion (13) of the retractable holder (12), the releasing part (19) being movable from a rest position to a release position, in response to a release force, for displaying said release portion (13),
wherein the handle includes an actuating mechanism (35) for producing said release force and the elongate shaft (5) includes means for transmitting said release force to the release part (19).

4. A surgical instrument (1) according to claim 2, wherein said retractable holder (12) includes a radically displaceable elongate release portion (13) with sloping release surfaces (14) and holding teeth (15) expending radically outward from a distal end of the elongate release portion (13) and configured to engage corresponding holding surfaces (16) of the distal compression ring (2A) and,
wherein the releasing part (19) is movable from a proximal rest position in a distal direction, in response to a compressive release force, to push against the sloping release surfaces (14) of the elongate release portion for misplacing said release portion to retract the holding teeth from the distal compression ring.

5. A surgical instrument (1) according to any one of the preceding claims, wherein the anvil (7) is detachable and comprises a tapered dilating portion (18) having a diameter which increases distally, said dilating portion (18) being suitable to dilate tissue pierced by the anvil.

6. A surgical instrument (1) according to any one of the preceding claims, wherein the actuating member (8) includes a cutting edge (38) movable from a proximal rest position in distal direction, in response to a compressive cutting force, for cutting proximal and distal tissue compressed between the proximal and distal compression rings (2A, 2B) to create the anastomotic orifice,
wherein the handle (6) includes an actuating mechanism for producing said compressive cutting force and the elongate shaft (5) includes means for transmitting said compressive cutting force to the cutting edge (38).

7. A surgical instrument (1) according to the previous claim, wherein the anvil (7) comprises a cut washer (39) providing a cutting surface for the cutting edge (38) to break through in order to complete the cut.

8. A surgical instrument (1) according to the previous claim, wherein the cut washer (39) includes a hooding part (40) projecting radially outward from the anvil (7) and being configured to hold the distal compression ring (2A) for connecting it to the anvil, wherein the cutting edge (38) is configured to cut away said projecting holding part (40) for disconnecting the distal compression ring (2A) from the anvil (7) and enabling the anvil to be withdrawn in proximal direction through the center openings of the anastomotic ring device.

9. A surgical instrument (1) according to any one of the preceding claims, wherein the actuating member (8) includes a needle applier (24) configured to hold a group of needles (36) or staples for piercing and fixating the tissue held between the distal and proximal compression ring,
wherein said needle applier (24) is movable from a proximal rest position in distal direction, in response to a compressive needle force, for pushing the group of needles (36) or staples into the anastomotic ring device,
wherein the handle (6) includes an actuating mechanism for producing said compressive needle force and the elongate shaft (5) includes means for transmitting said compressive needle force to the needle applier (24).

10. A surgical instrument (1) according claim 9, wherein said retention element (2C) defines a seat (28) for receiving a group of needles (36) or staples such that, in response to a distal movement of said actuating part (24), the snapper portion (2C) moves in snap engagement with the compression rings (2A, 2B) and pushes contemporaneously the group of needles (36) or staples in the anastomotic ring device piercing the tissue held between the proximal and distal compression rings.

11. A surgical instrument (1) according to any one of the preceding claims, wherein the elongate shaft (5) includes:
- an outer tube (21), a distal end of the outer tube defining a seat (22) for receiving the proximal compression ring (2A) and a proximal end of the outer tube being connected with a handle housing (6) enveloping the actuating mechanism of the surgical instrument,
- a substantially tubular actuating rod (23) slidingly arranged inside the outer tube (21), a distal end of the actuating rod defining a seat (24) for the retention element (2C) of the anastomotic ring device (2) and a proximal end (25) of the actuating rod reaching inside the handle housing (6) and being elastically biased in proximal direction in contact against an actuating trigger (27) suitable for pushing the actuating rod (23) in distal direction,
- an approximating rod (31) slidingly received inside the outer tube (21), a distal end of the approximating rod (31) defining said second connecting part (17) and a proximal end of said approximating rod reaching inside the handle housing (6) and being connected with an approximating knob (32) suitable for translating the approximating rod (31) in proximal direction.

12. A surgical instrument (1) according to the preceding claim, wherein the elongate shaft (5) includes a substantially tubular releasing rod (34) arranged slidingly inside the outer tube (21), a distal end of the releasing rod defining said releasing part (19) and a proximal end of the releasing rod extending inside the handle housing (6) and being connected with a releasing slider (35) suitable for pushing the releasing rod in distal direction in order to retract the retractable holder (12).

13. A surgical instrument (1) according to any one of claims 11 to 12, wherein said actuating trigger (27) includes a hinged trigger lever and the approximating knob (32) engages the rotatably locked proximal end of the approximating rod (31) by means of a threaded connector such that a rotation of the knob (32) causes the approximating rod to translate along the threaded connector.

14. A surgical instrument (1) according to claims 12 or 13, wherein said manual release slider (35) includes a longitudinally adjustable sliding button directly connected with the proximal end of the releasing rod (34).

15. A surgical instrument (1) according to any one of the preceding claims, wherein the first connecting part (9) of the anvil includes a proximal end having a trocar formed or connected on it, suitable to pierce through a tissue layer.

16. A surgical instrument (1) according to any one of the preceding claims, wherein the second connecting part (17) includes a distal end having a trocar or a sharp tip formed or connected on it, suitable to piece through a tissue layer.

17. A surgical instrument (1) according to any one of the preceding claims, wherein the elongate insertion shaft comprises a flexible portion to allow access through a curved lumen and wherein the rods housed inside the elongate shaft for transmitting the approximation, actuation, release and cutting movements comprise flexible portions in order to allow the flexion of the insertion shaft.

## Patentansprüche

1. Chirurgisches Instrument (1) zum Implantieren einer Anastomoseringvorrichtung (2), umfassend:
einen Amboss (7), der einen ersten Verbindungsteil (9) und einen Sitz (15) zum Aufnehmen eines distalen Kompressionsrings (2A) der Anastomoseringvorrichtung (2) enthält; und
ein Betätigungselement (8), umfassend:
einen oder mehrere Sitze (22) zum Aufnehmen eines proximalen Kompressionsrings (2B) und eines Rückhalteelements (2C) der Anastomoseringvorrichtung (2); und
einen zweiten Verbindungsteil (17), der so ausgeführt ist, dass er mit dem ersten Verbindungsteil (9) in Eingriff gelangt, um den Amboss (7) mit dem Betätigungselement (8) zu verbinden, wobei der zweite Verbindungsteil (17) in Reaktion auf eine Zugkraft von einer distalen Position in proximaler Richtung beweglich ist, um den Amboss (7) dem Betätigungselement (8) anzunähern; und
einen Betätigungsteil (24), der so ausgeführt ist, dass er auf das Rückhalteelement (2C) der Anastomoseringvorrichtung (2) einwirkt, wobei der Betätigungsteil (24) in Reaktion auf eine Kompressionskraft von einer proximalen Ruheposition in einer distalen Richtung beweglich ist, um das Rückhalteelement (2C) in Richtung zu dem distalen Kompressionsring (2A) und in den Eingriff mit demselben zu bewegen, um die Kompressionsringe zu verbinden;
einen Griff (6), der einen Betätigungsmechanismus (32, 33, 25, 26, 27) enthält, um die Annäherungszugkraft und die Betätigungskompressionskraft zu erzeugen; und
einen langgestreckten Schaft (5), der den Griff (6) mit dem Betätigungselement (8) verbindet, wobei der Schaft Mittel zum Übertragen der Annäherungszugkraft zu dem zweiten Verbindungsteil (17) und der Betätigungskompressionskraft zu dem Betätigungsteil (24) enthält.

2. Chirurgisches Instrument (1) nach Anspruch 1, wobei der erste Verbindungsteil (9) und der zweite Verbindungsteil (17) geeignet sind, mittels Schnappverbindung miteinander verbunden zu werden.

3. Chirurgisches Instrument nach Anspruch 1 oder 2, wobei der Amboss (7) einen zurückziehbaren Halter (12) umfasst, der so ausgeführt ist, dass er den distalen Kompressionsring (2A) hält, und einen Freigabeabschnitt (13) enthält, wobei ein Wechsel des Freigabeabschnitts (13) den zurückziehbaren Halter (12) veranlasst, sich radial nach innen zurückzuziehen und sich vom distalen Kompressionsring (2A) zu trennen, um zu ermöglichen, dass der Amboss (7) in proximaler Richtung durch die Zentralöffnungen der Anastomoseringvorrichtung (2) zurückgezogen wird,
wobei das Betätigungselement (8) einen Freigabeteil (19) umfasst, der so ausgeführt ist, dass er auf den Freigabeabschnitt (13) des zurückziehbaren Halters (12) einwirkt, wobei der Freigabeteil (19) in Reaktion auf eine Freigabekraft von einer Ruheposition zu einer Freigabeposition beweglich ist, um den Freigabeabschnitt (13) zu verschieben,
wobei der Griff einen Betätigungsmechanismus (35) zum Erzeugen der Freigabekraft enthält und der langgestreckte Schaft (5) Mittel zum Übertragen der Freigabekraft auf den Freigabeteil (19) enthält.

4. Chirurgisches Instrument (1) nach Anspruch 2, wobei der zurückziehbare Halter (12) einen radial verschiebbaren langgestreckten Freigabeabschnitt (13) mit schrägen Freigabeflächen (14) und Haltezähnen (15), die sich von einem distalen Ende des langgestreckten Freigabeabschnitts (13) radial nach außen erstrecken und so konfiguriert sind, dass sie mit entsprechenden Halteflächen (16) des distalen Kompressionsrings (2A) in Eingriff gelangen, enthält, und
wobei der Freigabeteil (19) in Reaktion auf eine Freigabekompressionskraft von einer proximalen Ruheposition in einer distalen Richtung beweglich ist, um gegen die schrägen Freigabeflächen (14) des langgestreckten Freigabeabschnitts zu drücken und den Freigabeabschnitt zu verschieben, um die Haltezähne vom distalen Kompressionsring zurückzuziehen.

5. Chirurgisches Instrument (1) nach irgendeinem der vorangehenden Ansprüche, wobei der Amboss (7) abnehmbar ist und einen abgeschrägten Erweiterungsabschnitt (18) umfasst, der einen Durchmesser aufweist, der distal zunimmt, wobei der Erweiterungsabschnitt (18) geeignet ist, vom Amboss durchstoßenes Gewebe zu erweitern.

6. Chirurgisches Instrument (1) nach irgendeinem der vorangehenden Ansprüche, wobei das Betätigungselement (8) eine Schneidkante (38) enthält, die in Reaktion auf eine Schneidekompressionskraft von einer proximalen Ruheposition in distaler Richtung beweglich ist, um proximal und distal Gewebe zu schneiden, das zwischen dem proximalen und distalen Kompressionsringen (2A, 2B) komprimiert ist, um die Anastomoseöffnung zu schaffen,
wobei der Griff (6) einen Betätigungsmechanismus zum Erzeugen der Schneidekompressionskraft enthält und der langgestreckte Schaft (5) Mittel zum Übertragen der Schneidekompressionskraft auf die Schneidkante (38) enthält.

7. Chirurgisches Instrument (1) nach dem vorangehenden Anspruch, wobei der Amboss (7) eine Schneidscheibe (39) umfasst, die eine Schneidfläche für den Durchstoß der Schneidkante (38) bereitstellt, um den Schnitt zu vollenden.

8. Chirurgisches Instrument (1) nach dem vorangehenden Anspruch, wobei die Schneidscheibe (39) einen Halteteil (40) enthält, der vom Amboss (7) radial nach außen hervorsteht und so ausgeführt ist, dass er den distalen Kompressionsring (2A) hält, um diesen mit dem Amboss zu verbinden, wobei die Schneidkante (38) so ausgeführt ist, dass sie den vorstehenden Halteteil (40) abschneidet, um den distalen Kompressionsring (2A) vom Amboss (7) zu trennen und dem Amboss zu ermöglichen, in proximaler Richtung durch die Zentralöffnungen der Anastomoseringvorrichtung zurückgezogen zu werden.

9. Chirurgisches Instrument (1) nach irgendeinem der vorangehenden Ansprüche, wobei das Betätigungselement (8) einen Nadelapplikator (24) enthält, der so ausgeführt ist, dass er eine Gruppe von Nadeln (36) oder Heftklammern zum Durchstoßen und Fixieren des zwischen dem distalen und dem proximalen Kompressionsring gehaltenen Gewebes hält,
wobei der Nadelapplikator (24) in Reaktion auf eine Nadelungskompressionskraft von einer proximalen Ruheposition in distaler Richtung beweglich ist, um die Gruppe von Nadeln (36) oder Heftklammern in die Anastomoseringvorrichtung zu drücken,
wobei der Griff (6) einen Betätigungsmechanismus zum Erzeugen der Nadelungskompressionskraft enthält und der langgestreckte Schaft (5) Mittel zum Übertragen der Nadelungskompressionskraft auf den Nadelapplikator (24) enthält.

10. Chirurgisches Instrument (1) nach Anspruch 9, wobei das Rückhalteelement (2C) einen Sitz (28) zum Aufnehmen einer Gruppe von Nadeln (36) oder Heftklammern definiert, so dass in Reaktion auf eine distale Bewegung des Betätigungsteils (24) der Schnappabschnitt (2C) sich in einen Schnappeingriff mit den Kompressionsringen (2A, 2B) bewegt und gleichzeitig die Gruppe von Nadeln (36) oder Heftklammern in die Anastomoseringvorrichtung drückt, die das zwischen dem proximalen und dem distalen Kompressionsring gehaltene Gewebe durchstoßen.

11. Chirurgisches Instrument (1) nach irgendeinem der vorangehenden Ansprüche, wobei der langgestreckte Schaft (5) enthält:
ein Außenrohr (21), wobei ein distales Ende des Außenrohres einen Sitz (22) zum Aufnehmen des proximalen Kompressionsrings (2A) definiert und ein proximales Ende des Außenrohrs mit einem Griffgehäuse (6) verbunden ist, das den Betätigungsmechanismus des chirurgischen Instruments umhüllt,
eine im Wesentlichen rohrförmige Betätigungsstange (23), die gleitend innerhalb des Außenrohres (21) angeordnet ist, wobei ein distales Ende der Betätigungsstange einen Sitz (24) für das Rückhalteelement (2C) der Anastomoseringvorrichtung (2) definiert und ein proximales Ende (25) der Betätigungsstange in das Griffgehäuse (6) reicht und in proximaler Richtung elastisch vorbelastet ist und an einem Betätigungsauslöser (27) anliegt, der geeignet ist, die Betätigungsstange (23) in distaler Richtung zu drücken,
eine Annäherungsstange (31), die gleitend innerhalb des Außenrohres (21) aufgenommen ist, wo ein distales Ende der Annäherungsstange (31) den zweiten Verbindungsteil (17) definiert und ein proximales Ende der Annäherungsstange in das Griffgehäuse (6) reicht und mit einem Annäherungsknopf (32) verbunden ist, der geeignet ist, die Annäherungsstange (31) in proximaler Richtung zu verschieben.

12. Chirurgisches Instrument (1) nach dem vorangehenden Anspruch, wobei der langgestreckte Schaft (5) eine im Wesentlichen rohrförmige Freigabestange (34) enthält, die gleitend innerhalb des Außenrohres (21) angeordnet ist, wobei ein distales Ende der Freigabestange den Freigabeteil (19) definiert und ein proximales Ende der Freigabestange in das Griffgehäuse (6) reicht und mit einem Freigabeschlitten (35) verbunden ist, der geeignet ist, die Freigabestange in distaler Richtung zu drücken, um den zurückziehbaren Halter (12) zurückzuziehen.

13. Chirurgisches Instrument (1) nach irgendeinem der Ansprüche 11 bis 12, wobei der Betätigungsauslöser (27) einen schwenkbaren Auslösehebel enthält und der Annäherungsknopf (32) mit dem drehfesten proximalen Ende der Annäherungsstange (31) mittels eines Schraubverbinders in Eingriff gelangt, so dass eine Drehung des Knopfes (32) die Annäherungsstange veranlasst, sich längs des Schraubverbinders zu verschieben.

14. Chirurgisches Instrument (1) nach einem der Ansprüche 12 oder 13, wobei der manuelle Freigabeschlitten (35) einen longitudinal einstellbaren Schlittenknopf enthält, der direkt mit dem proximalen Ende der Freigabestange (34) verbunden ist.

15. Chirurgisches Instrument (1) nach irgendeinem der vorangehenden Ansprüche, wobei der erste Verbindungsteil (9) des Ambosses ein proximales Ende enthält, das einen daran ausgebildeten oder damit verbundenen Trokar aufweist, der geeignet ist, durch eine Gewebeschicht zu stoßen.

16. Chirurgisches Instrument (1) nach irgendeinem der vorangehenden Ansprüche, wobei der zweite Verbindungsteil (17) ein distales Ende enthält, das einen Trokar oder eine scharfe Spitze enthält, der/die daran ausgebildet oder damit verbunden ist und geeignet ist, durch eine Gewebeschicht zu stoßen.

17. Chirurgisches Instrument (1) nach irgendeinem der vorangehenden Ansprüche, wobei der langgestreckte Einführungsschaft einen flexiblen Abschnitt umfasst, um einen Zugang durch ein gekrümmtes Lumen zu erlauben, wobei die innerhalb des langgestreckten Schaftes aufgenommenen Stangen zum Übertragen der Annäherungs-, Betätigungs-, Freigabe- und Schneidbewegungen flexible Abschnitte umfassen, um das Biegen des Einführungsschafts zu erlauben.

## Revendications

1. Instrument chirurgical (1) pour implanter un dispositif annulaire anastomotique, comprenant :
- une enclume (7) incluant une première partie de connexion (9) et un siège (15) pour recevoir une bague de compression distale (2A) dudit dispositif annulaire anastomotique (2), et
- un élément d'actionnement (8) comprenant :
- un ou plusieurs sièges (22) pour recevoir une bague de compression proximale (2B) et un élément de rétention (2C) dudit dispositif annulaire anastomotique (2),
- une seconde partie de connexion (17) configurée pour engager la première partie de connexion (9) pour connecter l'enclume (7) avec l'élément d'actionnement (8), dans laquelle la seconde partie de connexion (17) est mobile depuis une position distale en direction proximale en réponse à une force de traction, pour approcher l'enclume (7) vers l'élément d'actionnement (8), et
- une partie d'actionnement (24) configurée pour agir sur l'élément de rétention (2C) du dispositif annulaire anastomotique (2), la partie d'actionnement (24) étant mobile depuis une position de repos proximale dans une direction distale, en réponse à une force de compression, pour déplacer l'élément de rétention (2C) vers la bague de compression distale (2A) et en engagement avec celle-ci pour connecter les bagues de compression ;
- une poignée (6) incluant un mécanisme d'actionnement (32, 33, 25, 26, 27) pour produire ladite force d'approche par traction et ladite force d'actionnement par compression, et
- une tige allongée (5) qui connecte la poignée (6) avec l'élément d'actionnement (8), ladite tige incluant des moyens pour transmettre ladite force d'approche par traction à la seconde partie de connexion (17) et ladite force d'actionnement par compression à la partie d'actionnement (24).

2. Instrument chirurgical (1) selon la revendication 1, dans lequel ladite première partie de connexion (9) et ladite seconde partie de connexion (17) sont appropriées pour se connecter l'une à l'autre par encliquetage.

3. Instrument chirurgical (1) selon la revendication 1 ou 2, dans lequel l'enclume (7) comprend un organe de maintien rétractable (12) configuré pour maintenir ladite bague de compression distale (2A) et incluant une portion de libération (13), dans laquelle un remplacement de la portion de libération (13) amène l'organe de maintien rétractable (12) à se rétracter radialement vers l'intérieur et se déconnecter de la bague de compression distale (2A) pour permettre d'extraire l'enclume (7) en direction proximale via les ouvertures centrales du dispositif annulaire anastomotique (2),
dans lequel ledit élément d'actionnement (8) comprend une partie de libération (19) configurée pour agir sur la portion de libération (13) de l'organe de maintien rétractable (12), la partie de libération (19) étant mobile depuis une position de repos vers une position de libération, en réponse à une force de libération, pour déplacer ladite portion de libération (13),
dans lequel la poignée inclut un mécanisme d'actionnement (35) pour produire ladite force de libération, et la tige allongée (5) inclut des moyens pour transmettre ladite force de libération à la partie de libération (19).

4. Instrument chirurgical (1) selon la revendication 2, dans lequel ledit organe de maintien rétractable (12) inclut une portion de libération allongée (13) radialement déplaçable avec des surfaces de libération en pente (14) et des dents de maintien (15) s'étendant radialement vers l'extérieur depuis une extrémité distale de la portion de libération allongée (13) et configurées pour engager des surfaces de maintien correspondantes (16) de la bague de compression distale (2A), et
dans lequel la partie de libération (19) est mobile depuis une position de repos proximale dans une direction distale, en réponse à une force de libération par compression, pour pousser contre les surfaces de libération en pente (14) de la portion de libération allongée pour déplacer ladite portion de libération afin de rétracter les dents de maintien depuis la bague de compression distale.

5. Instrument chirurgical (1) selon l'une quelconque des revendications précédentes, dans lequel l'enclume (7) est détachable et comprend une portion de dilatation effilée (18) ayant un diamètre qui augmente en direction distale, ladite portion de dilatation (18) étant appropriée pour dilater les tissus percés par l'enclume.

6. Instrument chirurgical (1) selon l'une quelconque des revendications précédentes, dans lequel l'élément d'actionnement (8) inclut un bord de coupe (38) mobile depuis une position de repos proximale dans une direction radiale, en réponse à une force de coupe par compression, pour couper les tissus proximaux et distaux comprimés entre la bague de compression proximale et la bague de compression distale (2A, 2B) pour créer l'orifice anastomotique,
dans lequel la poignée (6) inclut un mécanisme d'actionnement pour produire ladite force de coupe par compression et la tige allongée (5) inclut des moyens pour transmettre ladite force de coupe par compression au bord de coupe (38).

7. Instrument chirurgical (1) selon la revendication précédente, dans lequel l'enclume (7) comprend une rondelle de découpe (39) présentant une surface de coupe pour la traversée du bord de coupe (38) afin de terminer la coupe.

8. Instrument chirurgical (1) selon la revendication précédente, dans lequel la rondelle de coupe (39) inclut une partie de maintien (40) qui se projette radialement vers l'extérieur depuis l'enclume (7) et qui est configurée pour maintenir la bague de compression distale (2A) et la connecter à l'enclume, dans lequel le bord de coupe (38) est configuré pour enlever par découpe ladite partie de maintien en projection (40) et déconnecter la bague de compression distale (2A) vis-à-vis de l'enclume (7) et permettre d'extraire l'enclume en direction proximale à travers les ouvertures centrales du dispositif annulaire anastomotique.

9. Instrument chirurgical (1) selon l'une quelconque des revendications précédentes, dans lequel l'élément d'actionnement (8) inclut un applicateur d'aiguilles (24) configuré pour tenir un groupe d'aiguilles (36) ou d'agrafes pour percer et fixer les tissus maintenus entre la bague de compression distale et la bague de compression proximale,
dans lequel ledit applicateur d'aiguilles (24) est mobile depuis une position de repos proximale en direction distale, en réponse à une force de compression destinée aux aiguilles, afin de pousser le groupe d'aiguilles (36) ou d'agrafes dans le dispositif annulaire anastomotique,
dans lequel la poignée (6) inclut un mécanisme d'actionnement pour produire ladite force de compression destinée aux aiguilles, et la tige allongée (5) inclut des moyens pour transmettre ladite force de compression destinée aux aiguilles vers l'applicateur d'aiguilles (24).

10. Instrument chirurgical (1) selon la revendication 9, dans lequel ledit élément de rétention (2C) définit un siège (28) pour recevoir un groupe d'aiguilles (36) ou d'agrafes de telle façon que, en réponse à un mouvement distal de ladite partie d'actionnement (24), la portion d'encliquetage (2C) se déplace en engagement en encliquetage avec les bagues de compression (2A, 2B) et pousse simultanément le groupe d'aiguilles (36) ou d'agrafes dans le dispositif annulaire anastomotique en perçant les tissus maintenus entre la bague de compression proximale et la bague de compression distale.

11. Instrument chirurgical (1) selon l'une quelconque des revendications précédentes, dans lequel la tige allongée (5) inclut :
- un tube extérieur (21), une extrémité distale du tube extérieur définissant un siège (22) pour recevoir la bague de compression proximale (2A) et une extrémité proximale du tube extérieur étant connectée à un boîtier de poignée (6) qui enveloppe le mécanisme d'actionnement de l'instrument chirurgical,
- une tige d'actionnement sensiblement tubulaire (23) agencée en coulissement à l'intérieur du tube extérieur (21), une extrémité distale de la tige d'actionnement définissant un siège (24) pour l'élément de rétention (2C) du dispositif annulaire anastomotique (2) et une extrémité proximale (25) de la tige d'actionnement atteignant l'intérieur du boîtier de poignée (6) et étant élastiquement sollicitée en direction proximale en contact contre un déclencheur d'actionnement (27) qui convient pour pousser la tige d'actionnement (23) en direction distale,
- une tige d'approche (31) reçu en coulissement à l'intérieur du tube extérieur (21), une extrémité distale de la tige d'approche (31) définissant ladite seconde partie de connexion (17) et une extrémité proximale de ladite tige d'approximation atteignant l'intérieur du boîtier de poignée (6) et étant connecté avec un bouton d'approche (32) approprié pour mettre la tige d'approximation (31) en translation en direction proximale.

12. Instrument chirurgical (1) selon la revendication précédente, dans lequel la tige allongée (5) inclut une tige de libération sensiblement tubulaire (34) agencée en coulissement à l'intérieur du tube extérieur (21), une extrémité distale de la tige de libération définissant ladite partie de libération (19) et une extrémité proximale de la tige de libération s'étendant à l'intérieur du boîtier de poignée (6) et étant connectée à un coulisseau de libération (35) approprié pour pousser la tige de libération en direction distale afin de rétracter l'organe de maintien rétractable (12).

13. Instrument chirurgical (1) selon l'une quelconque des revendications 11 à 12, dans lequel ledit déclencheur d'actionnement (27) inclut un levier de déclenchement articulé et le bouton d'approche (32) engage extrémité proximale, bloquée en rotation, de la tige d'approche (31) au moyen d'un connecteur à pas de vis de telle façon qu'une rotation du bouton (32) amène la tige d'approche à se déplacer en translation le long du connecteur à pas de vis.

14. Instrument chirurgical (1) selon la revendication 12 ou 13, dans lequel ledit coulisseau de libération manuel (35) inclut un bouton coulissant, ajustable longitudinalement, directement connecté à l'extrémité proximale de la tige de libération (34).

15. Instrument chirurgical (1) selon l'une quelconque des revendications précédentes, dans lequel la première partie de connexion (9) de l'enclume inclut une extrémité proximale comportant un trocart, formé sur elle-même ou connecté à elle-même, approprié pour percer à travers une couche de tissu.

16. Instrument chirurgical (1) selon l'une quelconque des revendications précédentes, dans lequel la seconde partie de connexion (17) inclut une extrémité distale ayant un trocart ou un embout pointu formé sur elle-même ou connecté à elle-même, approprié pour percer à travers une couche de tissu.

17. Instrument chirurgical (1) selon l'une quelconque des revendications précédentes, dans lequel la tige d'insertion allongée comprend une portion flexible pour permettre un accès via un lumen incurvé, et dans lequel les tiges abritées à l'intérieur de la tige allongée pour transmettre les mouvements d'approche, d'actionnement, de libération et de coupe comprennent des portions flexibles afin de permettre la flexion de la tige d'insertion.
